# FASCICULE DE BREVET EUROPEEN

(11) **EP 0 515 540 B1**
(45) Date de publication et mention de la délivrance du brevet: **06.07.1994**
(21) Numéro de dépôt: 91905054.2
(22) Date de dépôt: 20.02.1991
(51) Int. Cl.: C07C 229/34, C07C 227/00, C07D 305/14, A61K 31/335

(54) **DERIVE DE LA BETA-PHENYLISOSERINE, SA PREPARATION ET SON EMPLOI**
BETA-PHENYLISOSERINDERIVATE, IHRE HERSTELLUNG UND IHRE ANWENDUNG
DERIVATIVE OF BETA-PHENYLISOSERINE, PREPARATION AND UTILISATION THEREOF

(30) Priorité: 21.02.1990 FR 9002099
(43) Date de publication de la demande: 02.12.1992
(73) Titulaire: RHONE-POULENC RORER S.A., 92160 Antony (FR)
(72) Inventeur: DUCHESNE, Jean-Pierre, F-69007 Lyon (FR); MULHAUSER, Michel, F-69130 Ecully (FR)
(74) Mandataire: Pilard, Jacques
(86) Numéro de dépôt international: FR9100132
(87) Numéro de publication internationale: WO9113053

(56) Documents cités:
- EP-A- 0 336 840
- EP-A- 0 336 841
- EP-A- 0 400 971
- Journal of the Chemical Society, Chemical Communications, No. 18, 1988, Letchworth, GB; SUNGGAK KIM et al.: "A convenient method for beta-lactam formation from beta-amino acids using diphenylphosphinic chloride"
- Journal of Organic Chemistry, vol. 51, No. 1, 10 janvier 1986, Easton, US; J.-N. DENIS et al.: "An efficient, enantioselective synthesis of the taxol side chain", p. 46-50
- Bulletin Korean Chemical Society, vol. 9, No. 3, 22 mars 1988, Seoul; SUNGGAK KIM et al.: "A new method for beta-lactam formation from beta-amino acids using benzotriazol-1-yloxytris (dimethylamino) phosphonium hexafluorophosphate", p. 189-190
- Archiv der Pharmazie, vol. 307, 1974, Weinheim, DE; E. KAMANDI et al.: "Die Synthese von beta-Phenyl-isoserinen durch Ammonolyse von beta-Phenylglycidestern, I", pages 871-878
- Archiv der Pharmazie, vol. 308, 1975, Weinheim, DE; E. KAMADI et al.: "Die Synthese von beta-Phenyl-isoserinen durch Ammonolyse von beta-Phenylhlycidestern, II", p. 135-141
- Journal of the American Chemical Society, vol. 110, No. 19, 14 septembre 1988, Gaston, PA, US; R.A. HOLTON et al.: "A synthesis of taxusin", p. 6558-6560
- Journal of the American Chemical Society, vol. 110, No. 17, 17 août 1988, Gaston, PA, US; J.-N DENIS et al.: "A highly efficient, practical approach to natural taxol", pages 5917-5919

## Description

La présente invention concerne un nouveau dérivé de la β-phénylisosérine de formule : 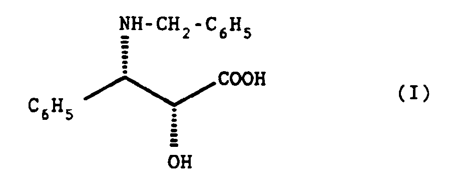
éventuellement sous forme de sel ou d'ester, sa préparation et son emploi.

D'après E. Kamandi et coll., Arch. Pharmaz., 307, 871-878 (1974), il est connu de préparer la thréo β-phénylisosérine par action de l'ammoniac sur un ester de l'acide cis-β-phénylglycidique suivie de l'action de la barite, afin d'éviter la racémisation, sur la β-phénylisosérine-amide intermédiairement obtenue.

D'après E. Kamandi et coll., Arch. Pharmaz., 308, 135-141 (1975), l'action de la benzylamine sur un ester de l'acide trans-β-phénylglycidique conduit à la formation de l'érythro-N,N'-dibenzyl-β-phénylisosérine-amide qui, par hydrogénation en présence de palladium sur charbon, conduit à l'érythro β-phénylisosérine-N-benzylamide.

Selon l'invention, le produit de formule (I) peut être obtenu par action de la benzylamine sur l'acide cis-β-phényl-glycidique-(2R,3R), de préférence sous forme d'un sel de métal alcalin tel que le sel de potassium ou d'un sel avec une base azotée telle que la benzylamine en opérant dans l'eau à une température généralement comprise entre 40 et 100°C.

Généralement, on utilise 1 à 40 moles de benzylamine par mole d'acide cis-β-phénylglycidique.

Le produit de formule (I), de préférence sous forme de sel de métal alcalin, tel que le sel de potassium, ou sous forme de sel avec une base azotée telle que la benzylamine, est obtenu après concentration à sec de la solution obtenue.

Le produit de formule (I) ainsi obtenu peut être transformé en ester de la β-phénylisosérine-(2R,3S) par action d'un alcool en milieu acide suivie de l'hydrogénation catalytique, de préférence en présence de palladium sur charbon.

L'ester de la β-phénylisosérine-(2R,3S) est particulièrement utile pour préparer les dérivés du taxane de formule générale : 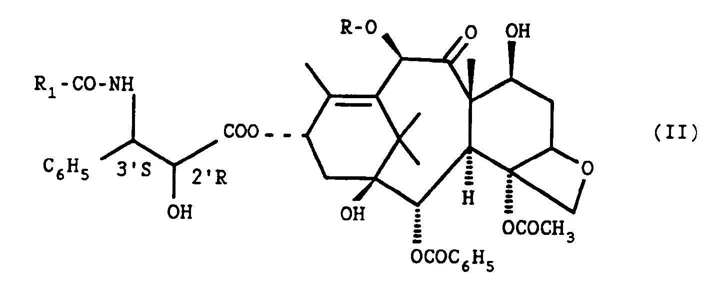
dans laquelle R représente un atome d'hydrogène ou un radical acétyle et R₁ représente un radical phényle ou t.butoxy, selon le procédé décrit dans les demandes de brevets européens EP 0 336 840 ou EP 0 336 841.

L'acide cis-β-phénylglycidique-(2R, 3R) peut être préparé dans les conditions décrites par J-N. Denis et coll., J. Org. Chem., 51, 46-50 (1986).

La présente invention concerne également le produit de formule générale (II) lorsqu'il est obtenu par un procédé mettant en oeuvre le produit de formule (I).

La présente invention concerne également les compositions pharmaceutiques contenant un produit de formule générale (II) lorsqu'il est obtenu par un procédé mettant en oeuvre le produit de formule (I), en association avec tout autre produit pharmaceutiquement acceptable qu'il soit inerte ou physiologiquement actif.

Ces compositions peuvent être présentées sous toute forme appropriée à la voie d'administration prévue. La voie parentérale est la voie d'administration préférentielle et notamment la voie intraveineuse.

Les compositions pour administration parentérale peuvent être des solutions stériles aqueuses ou non aqueuses, des suspensions ou des émulsions. Comme solvant ou véhicule, on peut employer le propylèneglycol, les huiles végétales, en particulier l'huile d'olive, et les esters organiques injectables, par exemple l'oléate d'éthyle. Ces compositions peuvent également comprendre des adjuvants, en particulier des agents mouillants, émulsifiants ou dispersants. La stérilisation peut se faire de plusieurs façons, par exemple à l'aide d'un filtre bactériologique, en incorporant à la composition des agents stérilisants, par irradiation ou par chauffage. Elles peuvent être également sous forme de compositions stériles qui peuvent être dissoutes ou dispersées dans de l'eau stérile ou tout autre milieu stérile injectable.

Ces compositions sont particulièrement utiles dans le traitement des leucémies aigues et des tumeurs solides.

Les exemples suivants, donnés à titre non limitatif, montrent comment l'invention peut être mise en pratique.

### EXEMPLE 1

Dans un réacteur, on introduit 18,2 g de sel de potassium de l'acide cis-β-phénylglycidique-(2R,3R) (0,09 mole), 65 cm3 d'eau et 180 cm3 de benzylamine. Le mélange est chauffé pendant 4 heures 30 minutes à 50°C. Après refroidissement, le mélange réactionnel est extrait avec 2 fois 200 cm3 de dichlorométhane. La phase aqueuse est concentrée à sec. On obtient ainsi 24,2 g de sel de potassium de la N-benzyl β-phénylisosérine-(2R,3S) dont le pouvoir rotatoire est [α]_{D}²⁰ = +38,4° (c = 3,6 ; eau).

La structure du produit est confirmée par les spectres infra-rouge, de masse et de résonance magnétique nucléaire.

### EXEMPLE 2

Dans un réacteur, on introduit 23,2 g du sel obtenu à l'exemple 1 (0,075 mole) et 260 cm3 de méthanol. La solution est saturée par de l'acide chlorhydrique gazeux sec. Après 3 heures d'agitation à une température voisine de 20°C, le chlorure de potassium qui a précipité est séparé par filtration. La solution est concentrée à sec et le résidu est lavé à l'acétone puis filtré. Le produit obtenu (23,84 g) est dissous dans 80 cm3 de méthanol. On ajoute 7 g de palladium sur charbon. Le mélange réactionnel est placé sous atmosphère d'hydrogène. Après quelques heures d'agitation à une température voisine de 20°C, l'absorption d'hydrogène est terminée. Après filtration et concentration du filtrat, on obtient 13,5 g de chlorhydrate de l'ester méthylique de la β-phénylisosérine-(2R,3S) dont le pouvoir rotatoire est [α]_{D}²⁰ = -18,5° (c = 1,2 ; eau).

### EXEMPLE 3

Dans un réacteur contenant 1 litre de dichlorométhane, on introduit 143 g de sel de l'acide cis-β-phénylglycidique-(2R,3R) avec l'a-méthylbenzylamine (0,5 mole). On refroidit la suspension à 15°C puis on ajoute en 10 minutes une solution de 33 g de potasse à 85 % (0,5 mole) dans 100 cm3 d'eau.

La phase aqueuse est séparée par décantation puis la phase organique est extraite par 50 cm3 d'eau.

Les phases aqueuses réunies sont lavées par 100 cm3 de chlorure de méthylène.

La solution aqueuse du sel de potassium est introduite dans un réacteur avec 54 g de benzylamine (0,5 mole). On chauffe à 80°C pendant 2 heures. Après refroidissement en 2 heures à une température voisine de 20°C, le mélange réactionnel est versé dans 3 litres d'eau glacée. On ajoute 170 cm3 d'une solution 1,5M d'acide sulfurique : le pH est alors de 7,7. Le produit qui précipite est séparé par filtration, lavé par 3 fois 600 cm3 d'eau et 300 cm3 d'acétone. Après séchage, on obtient 100,6 g de N-benzyl β-phénylisosérine-(2R,3S).

## Revendications

1. Nouveau dérivé de la β-phénylisosérine de formule : 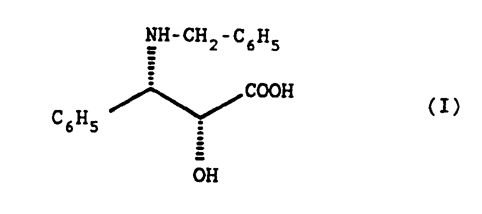 éventuellement sous forme de sel ou d'ester.

2. Procédé de préparation du produit selon la revendication 1 caractérisé en ce que l'on fait réagir la benzylamine sur l'acide cis-β-phénylglycidique-(2R,3R), de préférence sous forme de sel de métal alcalin ou de sel avec une base azotée en opérant dans l'eau et isole le produit obtenu que l'on transforme éventuellement en sel ou en ester.

3. Utilisation du produit selon la revendication 1 pour préparer la β-phénylisosérine-(2R,3S), de préférence sous forme d'ester, caractérisé en ce que l'on hydrogène catalytiquement le produit selon la revendication 1 de préférence sous forme d'ester.

4. Utilisation de la β-phénylisosérine-(2R,3S) obtenue selon le procédé de la revendication 3 pour la préparation, selon les méthodes connues, de dérivés du taxane de formule générale : 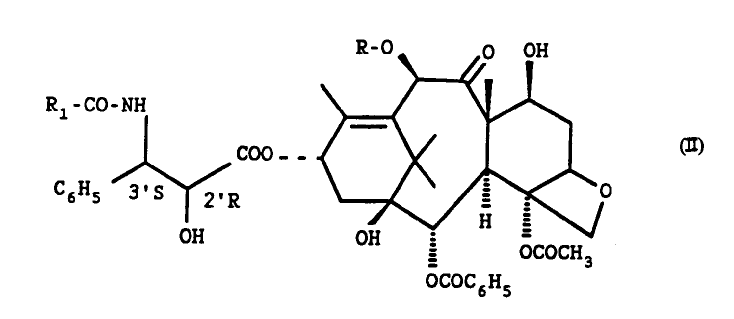 dans laquelle R représente un atome d'hydrogène ou un radical acétyle et R₁ représente un radical phényle ou t.butoxy.

5. Composition pharmaceutique caractérisée en ce qu'elle contient un dérivé obtenu selon la revendication 4 en association avec un ou plusieurs produits pharmaceutiquement acceptables qu'ils soient inertes ou physiologiquement actifs.

## Patentansprüche

1. Neues β-Phenylisoserinderivat der Formel: 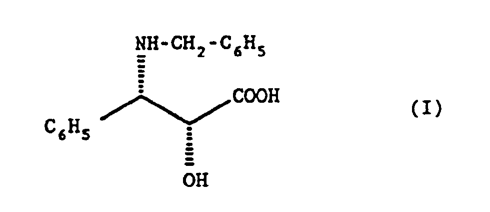 gegebenenfalls in Form eines Salzes oder Esters.

2. Verfahren zur Herstellung der Verbindung nach Anspruch 1, **dadurch gekennzeichnet,** daß man Benzylamin mit (2R,3R)-cis-β-Phenylglycidinsäure, bevorzugt in der Form des Alkalimetallsalzes oder des Salzes mit einer Stickstoffbase, in Wasser umsetzt und die erhaltene Verbindung isoliert, die man gegebenenfalls in ein Salz oder einen Ester überführt.

3. Verwendung der Verbindung nach Anspruch 1 zur Herstellung von (2R,3S)-β-Phenylisoserin, bevorzugt in der Form des Esters, **dadurch gekennzeichnet,** daß man die Verbindung nach Anspruch 1, bevorzugt in der Form des Esters, katalytisch hydriert.

4. Verwendung von (2R,3S)-β-Phenylisoserin, erhalten nach dem Verfahren nach Anspruch 3, zur Herstellung von Taxanderivaten der allgemeinen Formel 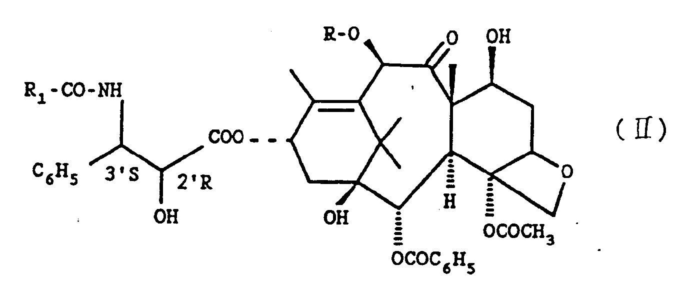 worin R für ein Wasserstoffatom oder einen Acetylrest steht, und R₁ für eine Phenyl- oder tert.-Butoxygruppe steht, nach bekannten Verfahren.

5. Pharmazeutisches Präparat, **dadurch gekennzeichnet,** daß es ein Derivat, erhalten nach Anspruch 4, zusammen mit einer oder mehreren pharmazeutisch verträglichen Verbindungen, die inert oder physiologisch aktiv sein können, enthält.

## Claims

1. New β-phenylisoserine derivative of formula: 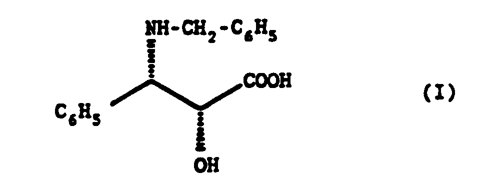 optionally in salt or ester form.

2. Process for the preparation of the product according to Claim 1, characterized in that benzylamine is reacted with (2R,3R)-cis-β-phenylglycidic acid, preferably in the form of an alkali metal salt or of a salt with a nitrogenous base, the operation being carried out in water, and the product obtained is isolated and is optionally converted into salt or into ester.

3. Use of the product according to Claim 1 for preparing (2R,3S)-β-phenylisoserine, preferably in ester form, characterized in that the product according to Claim 1 is hydrogenated catalytically, preferably in ester form.

4. Use of the (2R,3R)-β-phenylisoserine obtained according to the process of Claim 3, for preparing, by known methods, taxane derivatives of general formula: 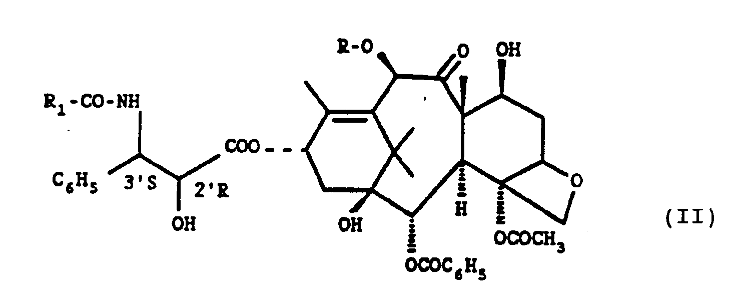 in which R denotes a hydrogen atom or an acetyl radical and R₁ denotes a phenyl or t-butoxy radical.

5. Pharmaceutical composition characterized in that it contains a derivative obtained according to Claim 4 in association with one or more pharmaceutically acceptable products, be they inert or physiologically active.
